Europäisches Patentamt

European Patent Office

Office européen des brevets

(19)

(11) **EP 1 056 485 B1**

(12) **EUROPÄISCHE PATENTSCHRIFT**

(45) Veröffentlichungstag und Bekanntmachung des Hinweises auf die Patenterteilung:
**17.07.2002   Patentblatt 2002/29**

(21) Anmeldenummer: **96942231.0**

(22) Anmeldetag: **23.12.1996**

(51) Int Cl.7: **A61L 27/00**

(86) Internationale Anmeldenummer:
**PCT/CH96/00461**

(87) Internationale Veröffentlichungsnummer:
**WO 98/28025 (02.07.1998 Gazette 1998/26)**

(54) **BIOAKTIVE OBERFLÄCHENSCHICHT FÜR KNOCHENIMPLANTATE**

BIOACTIVE SURFACE LAYER FOR BONE IMPLANTS

COUCHE SUPERFICIELLE BIOACTIVE POUR IMPLANTS OSSEUX

(84) Benannte Vertragsstaaten:
**AT BE CH DE DK ES FR GB IE LI LU NL PT SE**

(43) Veröffentlichungstag der Anmeldung:
**06.12.2000   Patentblatt 2000/49**

(73) Patentinhaber: **Stiftung, Robert Mathys H. C. Dr.
2544 Bettlach (CH)**

(72) Erfinder:
  • **PIVETEAU, Laurent-Dominique
    1700 Fribourg (CH)**
  • **GASSER, Beat
    3063 Ittigen (CH)**
  • **SCHLAPBACH, Louis
    3074 Muri (CH)**

(74) Vertreter: **Lusuardi, Werther Giovanni, Dr.
Dr. Lusuardi AG,
Kreuzbühlstrasse 8
8008 Zürich (CH)**

(56) Entgegenhaltungen:
**EP-A- 0 211 676          EP-A- 0 232 791
EP-A- 0 264 917          WO-A-94/25637
WO-A-96/24391          DE-A- 3 412 915**

  • **JOURNAL OF SOL-GEL SCIENCE AND
    TECHNOLOGY, Bd. 7, Nr. 1/02, 1.August 1996,
    Seiten 27-34, XP000620261 LI P ET AL:
    "BIOACTIVE CA10(PO4)6(OH)2-TIO2
    COMPOSITE COATING PREPARED BY
    SOL-GEL PROCESS"**

EP 1 056 485 B1

**Beschreibung**

Die Erfindung betrifft eine Oberflächenschicht für metallische Knochenimplantate gemäss dem Oberbegriff des Patentanspruchs 1. Metallische Knochenimplantate, insbesondere solche, die als Dauerimplantate vorgesehen sind, werden häufig mit einer bioaktiven Schicht überzogen, um eine dauerhafte und stabile Verankerung im oder auf dem Knochen zu erzielen. Alloplastische Biomaterialien werden als bioaktiv bezeichnet, wenn sie mit dem vitalen Knochengewebe eine festhaftende, chemische Verbindung eingehen. Die heute bekannten Oberflächenschichten, die vorwiegend durch Plasmabeschichtung erzeugt werden, weisen verschiedene Nachteile auf; dazu gehören die geringe Haftfestigkeit auf dem Substrat, Unsicherheiten in der Zusammensetzung (Kristallphase), wenig oder keine Variationsmöglichkeiten im Aufbau und der Zusammensetzung der Schicht, insbesondere auch der hohe Preis der Verfahren und der Produktionseinrichtungen.

Gemäss dem Stand der Technik sind bereits Mischungen aus Metalloxid (MeO) und Calciumphosphat (CaP) bekannt, ohne dass jedoch dafür spezifische Anwendungen auf dem medizinischen Gebiet offenbart wurden. Insbesondere finden sich keine konkreten Angaben bezüglich der Herstellung solcher Mischungen und ihrer Anwendung als Oberflächenschicht für chirurgische Implantate.

Aus der DE-A 34 12 915 ist ein Implantat bekannt, welches einen Grundkörper mit einem Oberflächenteil mit darin dispergierten Apatitteilchen aufweist und durch Schmelzen einer Mischung der Apatitteilchen im Grundkörper und Erstarrenlassen unter Anwendung der Fliehkraft erhalten wird. Nachteilig bei diesem Verfahren ist die Notwendigkeit den Grundkörper zu Schmelzen und der beim Erstarren anfallende kompakte Oberflächenteil mit darin dispergierten Apatitteilchen.

Hier will die Erfindung Abhilfe schaffen. Der Erfindung liegt die Aufgabe zugrunde, eine bioaktive Oberflächenschicht für Knochenimplantate auf einem metallischen Substrat zu schaffen, so dass keine Trennung von Schicht und Substrat auftritt.

Die Erfindung löst die gestellte Aufgabe mit einer bioaktiven Oberflächenschicht mit einer Dicke zwischen 0,01 µm und 5 mm für ein metallisches Knochenimplantat, wobei die Oberflächenschicht Calciumverbindungen (CaX) enthält bestehend aus kristallinem Calciumphosphat (CaP) und/oder Calciumapatiten (CaAp) und/oder Calciumcarbonat ($CaCO_3$), wobei für die Gesamtheit dieser drei in Partikelform als (CaX) vorliegenden Substanzen gilt:

$$100 \% \ (CaX) = \Sigma[x\% \ (CaP) + y\% \ (CaAp) + z\% \ (CaCO_3)],$$

wobei

x + y + z = 100, und
$0 \leq x \leq 100$
$0 \leq y \leq 100$
$0 \leq z \leq 100;$ und

wobei die Dimensionen der CaX-Partikel zwischen 2 nm und 4 mm liegen,

**dadurch gekennzeichnet, dass**
die Oberflächenschicht zusätzlich amorphes oder kristallines Metalloxid (MeO), mit Ausnahme von Aluminiumoxid enthält; und das molare Verhältnis CaX:MeO in der Oberflächenschicht in wählbarer Weise von unten nach oben zur freien Oberfläche hin zunimmt.

Die erfindungsgemässen Oberflächenschichten werden aus Mischungen von Metalloxiden und Calciumverbindungen hergestellt. Für die Beschichtung von Knochenimplantaten auf metallischen Substraten kommen Materialkomponenten, die als bioaktiv resp. bioinert bekannt sind, in Frage. Die in der nachfolgenden Beschreibung als Metalloxide und Calciumverbindungen bezeichneten Materialkomponenten sind als Sammelbegriffe zu verstehen. Die bioaktive Oberflächenschicht kann beispielsweise aus Titanoxid und Calciumhydroxylapatit bestehen. Die erfindungsgemässen Oberflächenschichten weisen zwischen metallischer (Substrat) und keramischer Phase (Schicht aus Oxid + Calciumverbindung) keine Trennung von Schicht und Substrat auf, womit ein Abreiben oder Abplatzen dieser keramischen Oberflächenschicht bei ungleichmässiger Beanspruchung verhindert wird.

Die Erfindung ist bei schraubenförmigen Implantaten von besonderem Interesse, sei es zur temporären Verankerung (z. B. Schanzsche Schrauben) oder zur permanenten Verankerung (z. B. Oralimplantate). Es ist auch möglich, diese Schichten dicht oder porös herzustellen.

Die Verankerung des mit einer erfindungsgemässen Oberflächenschicht versehenen Implantats erfolgt je nach Gestaltung nicht nur formschlüssig, sondern zu einem überwiegenden Teil auch kraftschlüssig. Dies aufgrund der chemischen Bindung der bioaktiven Oberflächenschicht mit dem anstossenden Knochengewebe, basierend auf der in der Oberflächenschicht integrierten Phase aus einer oder mehreren Calciumverbindungen.

Derartige Oberflächenschichten können durch nasschemische Verfahren (z.B. Sol-Gel Technik; Fällung) hergestellt werden, womit eine gute Reproduzierbarkeit und definierte Qualitätsmerkmale sichergestellt sind. Zudem sind diese Verfahren im Vergleich zu den bisher bekannten Methoden (CVD, PVD oder Plasmaspraying) verhältnismässig unkompliziert und preisgünstig.

Eine bevorzugte Weiterentwicklung der erfindungsgemässen Oberflächenschichten besteht darin, dass durch diese Herstellungsmethode verschiedene Varianten im Schichtaufbau möglich sind. Diese osteokonduktiven und bioaktiven Oberflächenschichten können dicht sein oder durch eine freiwählbare Porosität der spezifischen Anwendung angepasst werden. Ferner lässt sich über die ganze Schichtdicke die Zusammensetzung, z.B. das MeO:CaP-Verhältnis, auf optimale Weise bezüglich Bioaktivität beliebig verändern.

Die Erfindung und ihre Weiterentwicklung wird im folgenden anhand einer allgemeinen Beschreibung und zwei Ausführungsbeispielen näher erläutert.

## Allgemeine Beschreibung

Die zur Herstellung dieser bioaktiven Oberflächenschicht verwendeten Materialien sind ein oder mehrere Metalloxide (MeO) z.B. von Titan, Chrom, Niob, Tantal u. ä. oder Legierungen derselben in amorpher und/oder kristalliner Form sowie aus einer oder mehreren Calciumverbindungen (CaX) bestehend aus Anteilen von Calciumphosphaten (CaP) und/oder Calciumapatiten (CaAp) und/oder Calciumcarbonat ($CaCO_3$) in kristalliner Form. Dabei werden hier unter den Calciumphosphaten (CaP) Calciumhydroxylapatit (Hydroxylapatit, HA), $Ca_5(PO_4)_3OH$ und/oder $\beta$-Tri-Calciumphosphat ($\beta$-TCP), $Ca_3(PO_4)_2$ oder ähnliche verstanden. Unter dem Begriff "Calciumapatite" (CaAp) werden Carbonatapatit ($CO_3Ap$), $Ca_{10}(PO_4)_6CO_3$ und/oder Fluorapatit (FAp), $Ca_{10}(PO_4)_6F_2$ und/oder Chlorapatit (ClAp), $Ca_{10}(PO_4)_6Cl_2$ und/oder Oxyapatit (OAp), $Ca_{10}(PO_4)_6O$ verstanden. Die beschriebene Mischung dient der Beschichtung eines vorzugsweise metallischen, an der Oberfläche oxidierten oder nicht oxidierten Substrates. Das Konzentrationsverhältnis zwischen MeO und CaX kann über die ganze Schichtdicke konstant oder variabel gewählt werden. Vorzugsweise wird ein variables Verhältnis von MeO:CaX gewählt, das in der Nähe des metallischen Substrats einen höheren Wert aufweist. Die Dicke der Schicht kann zwischen 0,01 $\mu$m und 5 mm betragen.

Im weiteren können dichte oder poröse Schichten hergestellt werden. Für dichte Schichten hat sich eine Dicke zwischen 0,01 $\mu$m und 50 $\mu$m am besten bewährt; für poröse Schichten hingegen eine solche zwischen 10 $\mu$m und 3 mm. Vorzugsweise beträgt die Schichtdicke bei dichten Schichten zwischen 0,5 $\mu$m und 20 $\mu$m, bei porösen Schichten zwischen 100 $\mu$m und 1 mm. Die Grösse der CaX-Körner beträgt zweckmässigerweise 2 nm bis 4 mm.

Bei einer bevorzugten Ausführungsform besteht das Calciumphosphat (CaP) aus

- Calciumhydroxylapatit (HA), $Ca_5(PO_4)_3OH$ und/oder
- $\beta$-Tri-Calciumphosphat ($\beta$-TCP), $Ca_3(PO_4)_2$,

wobei $\Sigma[x_1\% \, (HA) + x_2\% \, (\beta\text{-TCP})] = x\%$; mit $0 \leq x_1, x_2 \leq x$; ist.

Das atomare Verhältnis Ca:P des (CaP) in der Oberflächenschicht liegt zweckmässigerweise zwischen 1:10 und 10:1, vorzugsweise zwischen 1:4 und 4:1.

Das atomare Verhältnis Ca:$CO_3$ des ($CaCO_3$) in der Oberflächenschicht liegt zweckmässigerweise zwischen 1:10 und 10:1, vorzugsweise zwischen 1:4 und 4:1.

Das atomare Verhältnis Ca:P des ($CO_3Ap$) in der Oberflächenschicht liegt zweckmässigerweise zwischen 1:10 und 10:1, vorzugsweise zwischen 1:4 und 4:1.

Für für den (CaP)-Anteil x gilt zweckmässigerweise die folgende Beziehung :

$$0 \leq x \leq 100, \text{ vorzugsweise } 50 \leq x \leq 100.$$

Bei einer bevorzugten Ausführungsform ist die bioaktive Oberflächenschicht porenfrei ausgebildet oder weist Poren mit einem Durchmesser von höchstens 1 $\mu$m auf.

## Ausführungsbeispiel 1

Man bereitet eine 1 m Tetrabutylorthotitanat ($C_{16}H_{36}O_4Ti$) - Lösung in Äthanol vor. Anschliessend wird ein Sol von Titan durch langsames Zufügen desselben Volumens einer Mischung von Wasser und Salpetersäure ($HNO_3$) in Äthanol hergestellt. Die Konzentrationen sind so zu wählen, dass die Verhältnisse Ti:$H_2O$ = 1:2 und Ti: $HNO_3$ = 100:1 betragen.

Ferner wird eine 0,5 m Calciumnitrat-Tetrahydrat-Lösung [$Ca(NO_3)\cdot 4H_2O$] in Äthanol vorbereitet. Dann wird Phosphorpentoxyd ($P_2O_5$) in Äthanol gelöst, um eine 1 m Phosphorester-Lösung

$$[\approx 50\% \; P(OR)_2(OH)O, \; \approx 50\% \; P(OR)(OH)_2O, \; < 1\% \; H_3PO_4;$$

wobei R = (CH$_2$CH$_3$)] zu bilden. Man mischt diese Lösung mit der Calciumnitratlösung, so dass das Ca:P-Verhältnis 10:6 beträgt. Diese Lösung wird dem zuvor hergestellten Titan-Sol langsam beigefügt.

Dabei können verschiedene Verhältnisse Ti:Ca gewählt werden, so dass beispielsweise die an das Substrat angrenzende Zone der Schicht eine höhere Konzentration an Titan und andererseits an der Oberfläche der Schicht eine höhere Konzentration der Calcium-Phosphor-Verbindung aufweist.

Diese Mischungen werden anschliessend mittels Dip-Coating auf das Titansubstrat abgeschieden. Diese Operation wird mehrmals mit Lösungen mit abnehmenden Ti:Ca-Verhältnissen wiederholt. Dabei wird das Implantat zwischen jeder Beschichtung während 5 Minuten auf 350°C erhitzt. Nach dem letzten Abscheidungsprozess wird das beschichtete Implantat während 5 Minuten auf 850°C erhitzt.

### Ausführungsbeispiel 2

Zitronensäure (C$_6$H$_8$O$_7$) wird in einer Konzentration von 4 m in Wasser gelöst. Anschliessend wird Tetrabutylorthotitanat (C$_{16}$H$_{36}$O$_4$Ti) und Acetylaceton (C$_5$H$_8$O$_2$) hinzugefügt. Das Verhältnis (C$_{16}$H$_{36}$O$_4$Ti):(C$_5$H$_8$O$_2$):(C$_6$H$_8$O$_7$) soll dabei 1:4:4 betragen. Im weiteren wird eine 1 m Calciumlösung (Ca) von Calciumacetat (C$_4$H$_6$CaO$_4$) in Wasser hergestellt. Nun wird Phosphorpentoxid (P$_2$O$_5$) in Äthanol gelöst, um eine 1m Phosphorester-Lösung

$$[\approx 50\% \; P(OR)_2(OH)O, \; \approx 50\% \; P(OR)(OH)_2O, \; < 1\% \; H_3PO_4;$$

$$\text{wobei R} = (CH_2CH_3)]$$

herzustellen. Dann wird diese Lösung mit der zuvor hergestellten Lösung aus Tetrabutylorthotitanat, Acetylaceton und Zitronensäure gemischt. Nun wird langsam die zuvor hergestellte Calciumacetat-Lösung beigefügt, wobei das Verhältnis Ca:P auf 10:6 festzulegen ist.

Auf diese Weise können verschiedene Ti:Ca-Verhältnisse gewählt werden, womit auch Schichten mit an das Substrat angrenzenden Zonen höherer Konzentration an Titan und andererseits an der Oberfläche höherer Konzentration der Calcium-Phosphor-Verbindung herstellbar sind.

Im folgenden wird die Zitronensäure-Lösung durch Hinzufügen von Äthylenglykol, und Verdampfung des Lösungsmittels polymerisiert.

Diese Mischungen sind anschliessend mittels Dip-Coating auf dem Titan-Substrat abzuscheiden. Dieser Vorgang wird mit Lösungen mit abnehmenden Ti:Ca-Verhältnissen wiederholt und dabei das Implantat zwischen jedem Beschichtungsvorgang während 5 Minuten auf 350° erhitzt. Nach Abschluss des letzten Beschichtungsvorgangs soll das beschichtete Implantat während 10 Minuten auf 850° erhitzt werden.

### Patentansprüche

1.    Bioaktive Oberflächenschicht mit einer Dicke zwischen 0,01 $\mu$m und 5 mm für ein metallisches Knochenimplantat, wobei die Oberflächenschicht Calciumverbindungen (CaX) enthält bestehend aus kristallinem Calciumphosphat (CaP) und/oder Calciumapatiten (CaAp) und/oder Calciumcarbonat (CaCO$_3$), wobei für die Gesamtheit dieser drei in Partikelform als (CaX) vorliegenden Substanzen gilt:

$$100\% \; (CaX) = \Sigma[x\% \; (CaP) + y\% \; (CaAp) + z\% \; (CaCO_3)],$$

wobei

x + y + z = 100, und
$0 \leq x \leq 100$
$0 \leq y \leq 100$
$0 \leq z \leq 100$; und

wobei die Dimensionen der CaX-Partikel zwischen 2 nm und 4 mm liegen,
**dadurch gekennzeichnet, dass**

die Oberflächenschicht zusätzlich amorphes oder kristallines Metalloxid (MeO), mit Ausnahme von Aluminiumoxid enthält; und das molare Verhältnis CaX:MeO in der Oberflächenschicht in wählbarer Weise von unten nach oben zur freien Oberfläche hin zunimmt.

**2.** Oberflächenschicht nach Anspruch 1, **dadurch gekennzeichnet, dass** das molare Verhältnis CaX:MeO in der Oberflächenschicht von unten nach oben zur freien Oberfläche hin kontinuierlich zunimmt.

**3.** Oberflächenschicht nach Anspruch 1 oder 2, **dadurch gekennzeichnet, dass** das Calciumphosphat (CaP) aus

- Calciumhydroxylapatit (HA), $Ca_5(PO_4)_3OH$ und/oder
- β-Tri-Calciumphosphat (β-TCP), $Ca_3(PO_4)_2$ besteht,

wobei

$$\Sigma[x_1\% \ (HA) + x_2\% \ (\beta\text{-TCP})] = x\%; \ \text{mit} \ 0 \leq x_1, x_2 \leq x; \ \text{ist}.$$

**4.** Oberflächenschicht nach einem der Ansprüche 1 bis 3, **dadurch gekennzeichnet, dass** es sich bei den Calciumapatiten um:

- Carbonatapatit $(CO_3Ap)$, $Ca_{10}(PO_4)_6CO_3$ und/oder
- Fluorapatit (FAp), $Ca_{10}(PO_4)_6F_2$ und/oder
- Chlorapatit (ClAp) $Ca_{10}(PO_4)_6Cl_2$ und/oder
- Oxyapatit (OAp) $Ca_{10}(PO_4)_6O$ handelt, wobei

$$\Sigma[y_1\%(CO_3Ap) + y_2\%(FAp) + y_3\%(ClAp) + y_4\%(OAp)] = y\%;$$

mit $\quad 0 \leq y_1, y_2, y_3, y_4 \leq y$ gilt.

**5.** Oberflächenschicht nach einem der Ansprüche 1 bis 4, **dadurch gekennzeichnet, dass** das atomare Verhältnis Ca:P des (CaP) in der Oberflächenschicht zwischen 1:10 und 10:1, vorzugsweise zwischen 1:4 und 4:1 liegt.

**6.** Oberflächenschicht nach einem der Ansprüche 1 bis 5, **dadurch gekennzeichnet, dass** das atomare Verhältnis $Ca:CO_3$ des $(CaCO_3)$ in der Oberflächenschicht zwischen 1:10 und 10:1, vorzugsweise zwischen 1:4 und 4:1 liegt.

**7.** Oberflächenschicht nach einem der Ansprüche 1 bis 6, **dadurch gekennzeichnet, dass** das atomare Verhältnis Ca:P des $(CO_3Ap)$ in der Oberflächenschicht zwischen 1:10 und 10:1, vorzugsweise zwischen 1:4 und 4:1 liegt.

**8.** Oberflächenschicht nach einem der Ansprüche 1 bis 7, **dadurch gekennzeichnet, dass** das atomare Verhältnis $CO_3:P$ des $(CO_3Ap)$ in der Oberflächenschicht zwischen 0:1 und 10:1, vorzugsweise zwischen 1: 1000 und 2:1 liegt.

**9.** Oberflächenschicht nach einem der Ansprüche 1 bis 8, **dadurch gekennzeichnet, dass** das molare Verhältnis $CaP:CO_3Ap$ in der Oberflächenschicht zwischen 1:0 und 0:1 und vorzugsweise zwischen 1:0 und 2:1 liegt.

**10.** Oberflächenschicht nach einem der Ansprüche 1 bis 9, **dadurch gekennzeichnet, dass** das molare Verhältnis $CaP:CaCO_3$ in der Oberflächenschicht zwischen 1:0 und 0:1 und vorzugsweise zwischen 1:0 und 2:1 liegt.

**11.** Oberflächenschicht nach einem der Ansprüche 1 bis 10, **dadurch gekennzeichnet, dass** das molare Verhältnis $CO_3Ap:CaCO_3$ in der Oberflächenschicht zwischen 1:0 und 0:1, vorzugsweise zwischen 10:1 und 1 :10 liegt.

**12.** Oberflächenschicht nach einem der Ansprüche 1 bis 11, **dadurch gekennzeichnet, dass** die bioaktive Oberflächenschicht porenfrei ausgebildet ist oder Poren mit einem Durchmesser von höchstens 1 μm aufweist.

**13.** Oberflächenschicht nach Anspruch 12, **dadurch gekennzeichnet, dass** die bioaktive, porenfreie Oberflächenschicht eine Dicke von 0,1 μm bis 50 μm, vorzugsweise von 0,5 μm bis 20 μm aufweist.

**14.** Oberflächenschicht nach einem der Ansprüche 1 bis 11, **dadurch gekennzeichnet, dass** die Dicke der bioaktiven Oberflächenschicht 10 μm bis 3 mm, vorzugsweise 100 μm bis 1 mm beträgt und dass die Oberflächenschicht mit Poren versehen ist.

**15.** Oberflächenschicht nach einem der Ansprüche 1 bis 14, **dadurch gekennzeichnet, dass** das metallische Substrat eine oxidierte Oberfläche aufweist.

**16.** Oberflächenschicht nach einem der Ansprüche 1 bis 15, **dadurch gekennzeichnet, dass** für den (CaP)-Anteil x die folgende Beziehung gilt:

$$0 \leq x \leq 100.$$

**17.** Oberflächenschicht nach Anspruch 16, **dadurch gekennzeichnet, dass** für den (CaP)-Anteil x die folgende Beziehung gilt:

$$50 \leq x \leq 100.$$

**Claims**

**1.** A bioactive surface layer of a thickness between 0,01 μm and 5 mm for a metal bone implant, the surface layer containing calcium compounds (CaX) consisting of crystalline calcium phosphate (CaP) and/or calcium apatites (CaAp) and/or calcium carbonate ($CaCO_3$), whereby for the sum of these three substances present in particulate form as (CaX) being defined by the equation:

$$100 \, \% \, (CaX) = \Sigma \, [x\% \, (CaP) + y\% \, (CaAp) + z\% \, (CaCO_3)]$$

whereby

$x + y + z = 100$, and
$0 \leq x \leq 100$
$0 \leq y \leq 100$
$0 \leq z \leq 100$; and

the dimensions of the CaX particles being between 2 nm and 4 mm,
**characterized in that**
the surface layer additionally contains amorphous or crystalline metal oxide (MeO) except for aluminum oxide and the molar ratio CaX : MeO in the surface layer selectively increases from bottom to top toward the free surface.

**2.** Surface layer as claimed in claim 1, wherein the molar ratio of CaX : MeO in the surface layer increases continuously from the bottom to top toward the free surface.

**3.** Surface layer as claimed in claim 1 or 2, wherein the calcium phosphate (CaP) consists of

- calcium hydroxyl apatite (HA), $Ca_5 \, (PO_4)_3OH$ and/or
- β - tricalcium phosphate (β - TCP), $Ca_3(PO_4)_2$,

wherein

$$\Sigma \, [x_1\% \, (HA) + x_2\% \, (\beta - TCP)] = x\%; \text{ and } 0 \leq x_1, x_2 \leq x.$$

**4.** Surface layer as claimed in one of the claims 1 to 3, wherein the calcium apatites are selected from the group consisting of

- carbonate apatite $(CO_3Ap)$, $Ca_{10}(PO_4)_6CO_3$ and/or
- fluoroapatite (FAp), $Ca_{10}(PO_4)_6F_2$ and/or
- chloroapatite (ClAp), $Ca_{10}(PO_4)_6Cl_2$ and/or
- oxyapatite (OAp), $Ca_{10}(PO_4)_6O$,

wherein

$$\Sigma \, [y_1\% \, (CO_3Ap) + y_2\% \, (FAp) + y_3\% \, (ClAp) + y_4\% \, (OAp) \, ] = y\%; \text{ and}$$

$$0 \le y_1, y_2, y_3, y_4 \le y.$$

5. Surface layer as claimed in one of the claims 1 to 4, wherein the atomic ratio Ca:P of (CaP) in the surface layer is between 1:10 and 10:1, preferably between 1:4 and 4:1.

6. Surface layer as claimed in one of the claims 1 to 5, wherein the atomic ration $Ca:CO_3$ of $(CaCO_3)$ in the surface layer is between 1:10 and 10:1, preferably between 1:4 and 4:1.

7. Surface layer as claimed in one of the claims 1 to 6, wherein the atomic ration Ca:P of $(CO_3Ap)$ in the surface layer is between 1:10 and 10:1, preferably between 1:4 and 4:1.

8. Surface layer as claimed in one of the claims 1 to 7, wherein the atomic ration $CO_3:P$ of the $(CO_3Ap)$ in the surface layer is between 0:1 and 10:1, preferably between 1:1000 and 2:1.

9. Surface layer as claimed in one of the claims 1 to 8, wherein the molar ratio $CaP:CO_3Ap$ in the surface layer is between 1:0 and 0:1, preferably between 1:0 and 2:1.

10. Surface layer as claimed in one of the claims 1 to 9, wherein the molar ratio $CaP:CaCO_3$ in the surface layer is between 1:0 and 0:1, preferably between 1:0 and 2:1.

11. Surface layer as claimed in one of the claims 1 to 10, wherein the molar ratio $CO_3Ap:CaCO_3$ in the surface layer is between 1:0 and 0:1, preferably between 10:1 and 1:10.

12. Surface layer as claimed in one of the claims 1 to 11, wherein the bioactive surface layer is free of pores or comprises pores of a diameter no more than 1 µm.

13. Surface layer as claimed in claim 12, wherein the thickness of the bioactive, pore-free surface layer is between 0,1 µm and 50 µm, preferably between 0,5 µm and 20 µm.

14. Surface layer as claimed in one of the claims 1 to 11, wherein the thickness of the bioactive surface layer is between 10 µm and 3 mm, preferably between 100 µm and 1 mm and wherein the surface layer is porous.

15. Surface layer as claimed in one of the claims 1 to 14, wherein the metal substrate comprises an oxidized surface.

16. Surface layer as claimed in one of the claims 1 to 15, wherein the following relation governs the (CaP) portion x:

$$0 \le x \le 100.$$

17. Surface layer as claimed in claim 16, wherein the following relation governs the (CaP) portion x:

$$50 \le x \le 100.$$

**Revendications**

1. Couche superficielle bioactive ayant une épaisseur comprise entre 0,01 μm et 5 mm, pour un implant métallique pour os, la couche superficielle contenant des composés de calcium (CaX) constitués de phosphate de calcium cristallin (CaP) et/ou d'apatites calciques (CaAp) et/ou de carbonate de calcium (CaCO$_3$),

$$100\% \, (CaX) = \Sigma \, [x\% \, (CaP) + y\% \, (CaAp) + z\% \, (CaCO_3)],$$

   où

   x + y + z = 100, et
   o $\leq$ x $\leq$ 100
   o $\leq$ y $\leq$ 100
   o $\geq$ z $\geq$ 100,

   valant pour la totalité de ces trois substances présentes sous forme particulaire comme (CaX), les dimensions des particules de CaX étant situées entre 2 nm et 4 mm,
   **caractérisée en ce que** la couche superficielle contient en supplément un oxyde métallique (MeO) amorphe ou cristallin, à l'exception d'oxyde d'aluminium, et **en ce que** le rapport molaire CaX/MeO dans la couche superficielle augmente d'une manière sélectionnable du bas vers le haut en direction de la surface libre.

2. Couche superficielle suivant la revendication 1, **caractérisée en ce que** le rapport molaire CaX/MeO dans la couche superficielle augmente continuellement du bas vers le haut en direction de la surface libre.

3. Couche superficielle suivant l'une des revendications 1 et 2, **caractérisée en ce que** le phosphate de calcium (CaP) est constitué

   - d'hydroxylapatite calcique (HA), Ca$_5$(PO$_4$)$_3$OH et/ou
   - de phosphate tricalcique β (β-TCP), Ca$_3$(PO$_4$)$_2$,

   où

$$\Sigma[x_1\% \, (HA) + x_2\% \, (\beta\text{-}TCP)] = x\%, \text{ avec } 0 \leq x_1, x_2 \leq x.$$

4. Couche superficielle suivant l'une des revendications 1 à 3, **caractérisée en ce que**, pour ce qui concerne les apatites calciques, il s'agit

   - de carbonate-apatite (CO$_3$Ap), Ca$_{10}$(PO$_4$)$_6$CO$_3$, et/ou
   - de fluoro-apatite (FAp), Ca$_{10}$(PO$_4$)$_6$F$_2$,et/ou
   - de chloro-apatite (ClAp) Ca$_{10}$(PO$_4$)$_6$Cl$_2$, et/ou
   - d'oxy-apatite (OAp), Ca$_{10}$(PO$_4$)$_6$O,

   où

$$\Sigma[y_1\% \, (CO_3Ap)) + y_2\% \, (FAp)] + y_3\% \, (ClAp) + y_4\% \, (OAp)] =$$

$$y\%, \text{ avec } 0 \leq y_1, y_2, y_3, y_4 \leq x.$$

5. Couche superficielle suivant l'une des revendications 1 à 4, **caractérisée en ce que** le rapport atomique Ca/P du (CaP) dans la couche superficielle se situe entre 1/10 et 10/1, de préférence entre 1/4 et 4/1.

6. Couche superficielle suivant l'une des revendications 1 à 5, **caractérisée en ce que** le rapport atomique Ca/CO$_3$ du (CaCO$_3$) dans la couche superficielle se situe entre 1/10 et 10/1, de préférence entre 1/4 et 4/1.

7. Couche superficielle suivant l'une des revendications 1 à 6, **caractérisée en ce que** le rapport atomique Ca/P du

(CO$_3$Ap) dans la couche superficielle se situe entre 1/10 et 10/1, de préférence entre 1/4 et 4/1.

8. Couche superficielle suivant l'une des revendications 1 à 7, **caractérisée en ce que** le rapport atomique CO$_3$/P du (CO$_3$Ap) dans la couche superficielle se situe entre 0/1 et 10/1, de préférence entre 1/1000 et 2/1.

9. Couche superficielle suivant l'une des revendications 1 à 8, **caractérisée en ce que** le rapport molaire Ca/P/ CO$_3$Ap dans la couche superficielle se situe entre 1/0 et 0/1 et de préférence entre 1/0 et 2/1.

10. Couche superficielle suivant l'une des revendications 1 à 9, **caractérisée en ce que** le rapport molaire CaP/CaCO$_3$ dans la couche superficielle se situe entre 1/0 et 0/1 et de préférence entre 1/0 et 2/1.

11. Couche superficielle suivant l'une des revendications 1 à 10, **caractérisée en ce que** le rapport molaire CO$_3$Ap/ CaCO$_3$ dans la couche superficielle se situe entre 1/0 et 0/1, de préférence entre 10/1 et 1/10.

12. Couche superficielle suivant l'une des revendications 1 à 11, **caractérisée en ce que** la couche superficielle bioactive est réalisée sans pores ou **en ce qu'**elle présente des pores ayant un diamètre au maximum de 1 μm.

13. Couche superficielle suivant la revendication 12, **caractérisée en ce que** la couche superficielle bioactive, sans pores, présente une épaisseur de 0,1 μm à 50 μm, de préférence de 0,5 μm à 20 μm.

14. Couche superficielle suivant l'une des revendications 1 à 11, **caractérisée en ce que** l'épaisseur de la couche superficielle bioactive est de 10 μm à 3 mm, de préférence de 100 μm à 1 mm et **en ce que** la couche superficielle est pourvue de pores.

15. Couche superficielle suivant l'une des revendications 1 à 14, **caractérisée en ce que** la substrat métallique présente une surface oxydée.

16. Couche superficielle suivant l'une des revendications 1 à 15, **caractérisée en ce que**, pour la fraction x de (CaP), l'équation suivante est valable :

$$0 \leq x \leq 100.$$

17. Couche superficielle suivant la revendication 16, **caractérisée en ce que**, pour la fraction x de (CaP), l'équation suivante est valable :

$$50 \leq x \leq 100.$$